# EUROPEAN PATENT SPECIFICATION

(11) **EP 1 904 484 B1**
(45) Date of publication and mention of the grant of the patent: **26.08.2009**
(21) Application number: 06791540.5
(22) Date of filing: 17.07.2006
(51) Int. Cl.: C07D 403/12, A61K 31/496

(54) **COMPOUNDS**
VERBINDUNGEN
COMPOSES

(30) Priority: 19.07.2005 GB 0514811
(43) Date of publication of application: 02.04.2008
(73) Proprietor: GLAXO GROUP LIMITED, Greenford, Middlesex UB6 ONN (GB)
(72) Inventor: ANCLIFF, Rachael, Ann, Stevenage Hertfordshire SG1 2NY (GB); BAMFORD, Mark, James GlaxoSmithKline, Harlow Essex CM19 5AW (GB); HODGSON, Simon, Teanby, Stevenage Hertfordshire SG1 2NY (GB); PARR, Christopher, Allan GlaxoSmithKline, Harlow Essex CM19 5AW (GB); PROCOPIOU, Panayiotis, Alexandrou, Stevenage Hertfordshire SG1 2NY (GB); WILSON, David, Matthew GlaxoSmithKline, Harlow Essex CM19 5AW (GB); WOODROW, Michael, Stevenage Hertfordshire SG1 2NY (GB)
(74) Representative: Priestley, Ruth Elizabeth
(86) International application number: PCT/EP2006/007034
(87) International publication number: WO 2007/009739

(56) References cited:
- WO-A-20/04035556

## Description

The present invention relates to compounds, processes for their preparation, compositions containing them and to their use in the treatment of various disorders, in particular inflammatory and/or allergic disorders of the respiratory tract.

Allergic rhinitis, pulmonary inflammation and congestion are medical conditions that are often associated with other conditions such as asthma, chronic obstructive pulmonary disease (COPD), seasonal allergic rhinitis and perennial allergic rhinitis. In general, these conditions are mediated, at least in part, by inflammation associated with the release of histamine from various cells, in particular mast cells.

Allergic rhinitis, also known as 'hay fever' affects a targe proportion of the population worldwide. There are two types of allergic rhinitis, seasonal and perennial. The clinical symptoms of seasonal allergic rhinitis, typically include nasal itching and irritation, sneezing and watery rhinorrhea which is often accompanied by nasal congestion. The clinical symptoms of perennial allergic rhinitis are similar except that nasal blockage may be more pronounced. Either type of allergic rhinitis may also cause other symptoms such as itching of the throat and/or eyes, epiphora and oedema around the eyes. The symptoms of allergic rhinitis may vary in intensity from the nuisance level to debilitating.

Allergic rhinitis and other allergic conditions are associated with the release of histamine from various cell types but particularly mast cells. The physiological effects of histamine are classically mediated by three receptor subtypes, termed H1, H2 and H3. H1 receptors are widely distributed throughout the CNS and periphery, and are involved in wakefulness and acute inflammation. H2 receptors mediate gastric acid secretion in response to histamine. H3 receptors are present on the nerve endings in both the CNS and periphery and mediate inhibition of neurotransmitter release [Hill et al., Pharmacol. Rev. 49:253-278 (1997)]. Recently, a fourth member of the histamine receptor family has been identified, termed the H4 receptor [Hough, Mol. Pharmacol. 59:415-419, (2001)]. Whilst the distribution of the H4 receptor appears to be restricted to cells of the immune and inflammatory systems, a physiological role for this receptor remains to be identified.

The activation of H1 receptors in blood vessels and nerve endings are responsible for many of the symptoms of allergic rhinitis, which include itching, sneezing, and the production of watery rhinorrhea. Antihistamine compounds, i.e. drugs which are selective H1 receptor antagonists such as chlorphenyramine and cetirizine, are effective in treating the itching, sneezing and rhinorrhea associated with allergic rhinitis, but are not effective against the nasal congestion symptoms [Aaronson, Ann. Allergy, 67:541-547, (1991)].

Histamine H3 receptors are expressed widely on both CNS and peripheral nerve endings and mediate the inhibition of neurotransmitter release. *In vitro* electrical stimulation of peripheral sympathetic nerves in isolated human saphenous vein results in an increase in noradrenaline release and smooth muscle contraction, which can be inhibited by histamine H3 receptor agonists [Molderings et al., Naunyn-Schmiedeberg's Arch. Pharmacol., 346:46-50, (1992); Valentine et al., Eur. J. Pharmacol., 366:73-78, (1999)]. H3 receptor agonists also inhibit the effect of sympathetic nerve activation on vascular tone in porcine nasal mucosa [Varty & Hey, Eur. J. Pharmacol., 452:339-345, (2002)]. *In vivo*, H3 receptor agonists inhibit the decrease in nasal airway resistance produced by sympathetic nerve activation [Hay et al., Arzneim-Forsch Drug Res., 48:881-888 (1998)]. Activation of Histamine H3 receptors in human nasal mucosa inhibits sympathetic vasoconstriction [Varty et al., Eur. J. Pharmacol., 484:83-89, (2004)]. Furthermore, H3 receptor antagonists in combination with histamine H1 receptor antagonists have been shown to reverse the effects of mast cell activation on nasal airway resistance and nasal cavity volume, an index of nasal congestion [Mcleod et al., Am. J. Rhinol., 13:391-399, (1999)], and further evidence for the contribution of H3 receptors to histamine-induced nasal blockage is provided by histamine nasal challenge studies performed on normal human subjects [Taylor-Clark et al., Br. J. Pharmacol., 1-8 (2005)].

WO2004/035556 describes certain substituted piperazines, (1,4)-diazepines and 2,5-diazabicycol[2.2.1]heptanes as histamine H1 and/or H3 antagonists or Histamine H3 reverse antagonists.

The present invention relates to compounds (or salts thereof) that are histamine H3 antagonists and/or inverse agonists. These compounds (or salts thereof) may be useful in the treatment of various disorders in particular inflammatory and/or allergic disorders, such as inflammatory and/or allergic disorders of the respiratory tract, for example allergic rhinitis, that are associated with the release of histamine from cells such as mast cells. Further, the compounds of the invention (or salts thereof) may show an improved profile over known H3 antagonists/inverse agonists in that they may possess one or more of the following properties:
(i) potent H3 antagonist/inverse agonist activity;
(ii) at least 100 fold more selective for the H3 receptor over the H1 receptor;
(iii) low CNS penetration;
(iv) improved bioavailability; and
(v) lower clearance and/or longer half-life in blood.

Compounds having such a profile may be orally effective, and/or capable of once daily administration and/or further may have an improve side effect profile compared with other existing therapies.

Thus the present invention provides, in a first aspect, a compound of formula (I): 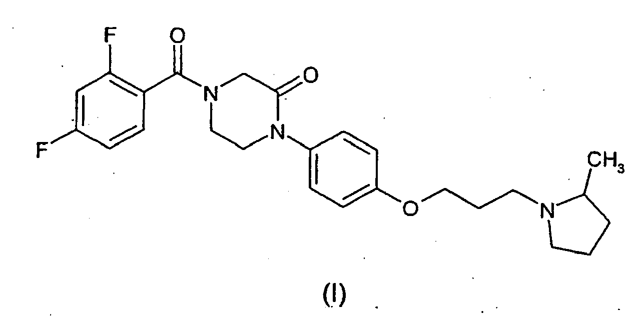 or a salt thereof, such as a pharmaceutically acceptable salt.

It will be appreciated that compounds of formula (I) possess an asymmetric carbon atom so that optical isomers e.g. enantiomers may be formed. The present invention encompasses all optical isomers of the compounds of formula (I) whether as individual isomers isolated to such as to be substantially free of the other isomer (i.e. pure) or as mixtures thereof. An individual isomer isolated such as to be substantially free of the other isomer (i.e. pure) may be isolated such that less than about 10%, particularly less than about 1%, for example less than about 0.1 % of the other isomer is present.

Thus the present invention includes the racemate 4-[(2,4-difluorophenyl)carbonyl]-1-[4-({3-[2-methyl-1-pyrrolidinyl]propyl}oxy)phenyl]-2-piperazinone, the R isomer 4-[(2,4-difluorophenyl)carbonyl]-1-[4-({3-[(2*R*)-2-methyl-1-pyrrolidinyl]propyl}oxy)phenyl]-2-piperazinone and the S isomer 4-[(2,4-difluorophenyl)carbonyl]-1-[4-({3-[(2*S*)-2-methyl-1-pyrrolidinyl]propyl}oxy)phenyl]-2-piperazinone and salts or solvates thereof.

It is to be further understood that the present invention covers the compounds of formula (I) as the free base and as salts thereof, such as pharmaceutically acceptable salts.

It is to be further understood that references hereinafter to a compound of formula (I) or the compound of the invention means a compound of formula (I) as the free base, or as a salt, or as a solvate.

The compounds of the present invention may be in the form of and/or may be administered as a pharmaceutically acceptable salt. Pharmaceutically acceptable salts include acid addition salts. For a review on suitable salts see Berge et al., J. Pharm. Sci., 66:1-19 (1977).

A pharmaceutically acceptable acid addition salt may be readily prepared by using a desired acid as appropriate. The salt may precipitate from solution and be collected by filtration or may be recovered by evaporation of the solvent. Typically a pharmaceutically acceptable acid addition salt can be formed by reaction of a compound of formula (I) with a suitable inorganic or organic acid (such as hydrobromic, hydrochloric, formic, sulfuric, nitric, phosphoric, succinic, maleic, acetic, fumaric, citric, tartaric, benzoic, p-toluenesulfonic, methanesulfonic or naphthalenesulfonic acid), optionally in a suitable solvent such as an organic solvent, to give the salt which is usually isolated for example by crystallisation and filtration. Thus, a pharmaceutically acceptable acid addition salt of a compound of formula (I) can be for example a hydrobromide, hydrochloride, formate, sulfate, nitrate, phosphate, succinate, maleate, acetate, fumarate, citrate, tartrate, benzoate, *p*-toluenesulfonate, methanesulfonate or naphthalenesulfonate salt.

Other non-pharmaceutically acceptable salts, e.g. oxalates or trifluoroacetates, may be used, for example in the isolation of compounds of the invention, and are included within the scope of this invention. The invention includes within its scope all possible stoichiometric and non-stoichiometric forms of the salts of the compounds of formula (I).

It will be appreciated that many organic compounds can form complexes with solvents in which they are reacted or from which they are precipitated or crystallized. These complexes are known as "solvates". For example, a complex with water is known as a "hydrate". Solvents with high boiling points and/or solvents with a high propensity to form hydrogen bonds such as water, xylene, N-methyl pyrrolidinone methanol may be used to form solvates. Methods for identification of solvates include, but are not limited to, NMR and microanalysis. Solvates of the compounds of the invention are within the scope of the invention.

The compounds of formula (I) may be In crystalline or amorphous form. Furthermore, some of the crystalline forms of the compounds of formula (I) may exist as polymorphs, which are included within the scope of the present invention. The most thermodynamically stable polymorphic forms of the compounds of formula (I) are of particular interest.

Polymorphic forms of the compounds of formula (I) may be characterized and differentiated using a number of conventional analytical techniques, including, but not limited to, X-ray powder diffraction (XRPD) patterns, infrared (IR) spectra, Raman spectra, differential scanning calorimetry (DSC), thermogravimetric analysis (TGA) and solid state nuclear magnetic resonance (NMR).

Certain compounds of formula (I) may exist in one of several tautomeric forms. It will be understood that the present invention encompasses all tautomers of the compounds of formula (I) whether as individual tautomers or as mixtures thereof.

It will be appreciated from the foregoing that included within the scope of the invention are all solvates, hydrates, complexes, tautomers/, optical isomers and polymorphic forms of the compounds of the invention and salts thereof.

The present invention also provides processes for the preparation of the compounds of formula (I) or salts thereof.

Thus, according to a first process A, a compound of formula (I) may be prepared by reacting a compound of formula (II) 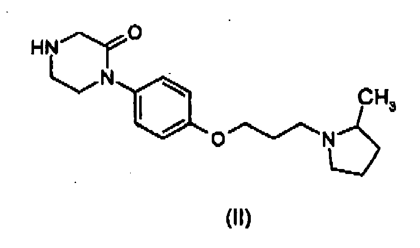 or a salt thereof with 2,4-difuorobenzoic acid (which is commercially available from Aldrich, for example).

The reaction may be carried out in the presence of a suitable base such as triethylamine and a suitable coupling agent such as *O*-(benzotriazol-1-yl)-*N*,*N*,*N'*,*N'*-tetramethyluronium tetrafluoroborate (TBTU), in an appropriate solvent such as dichloromethane or *N,N-*dimethylformamide.

Compounds of formula (II) may be prepared in accordance with the following or similar reaction scheme:

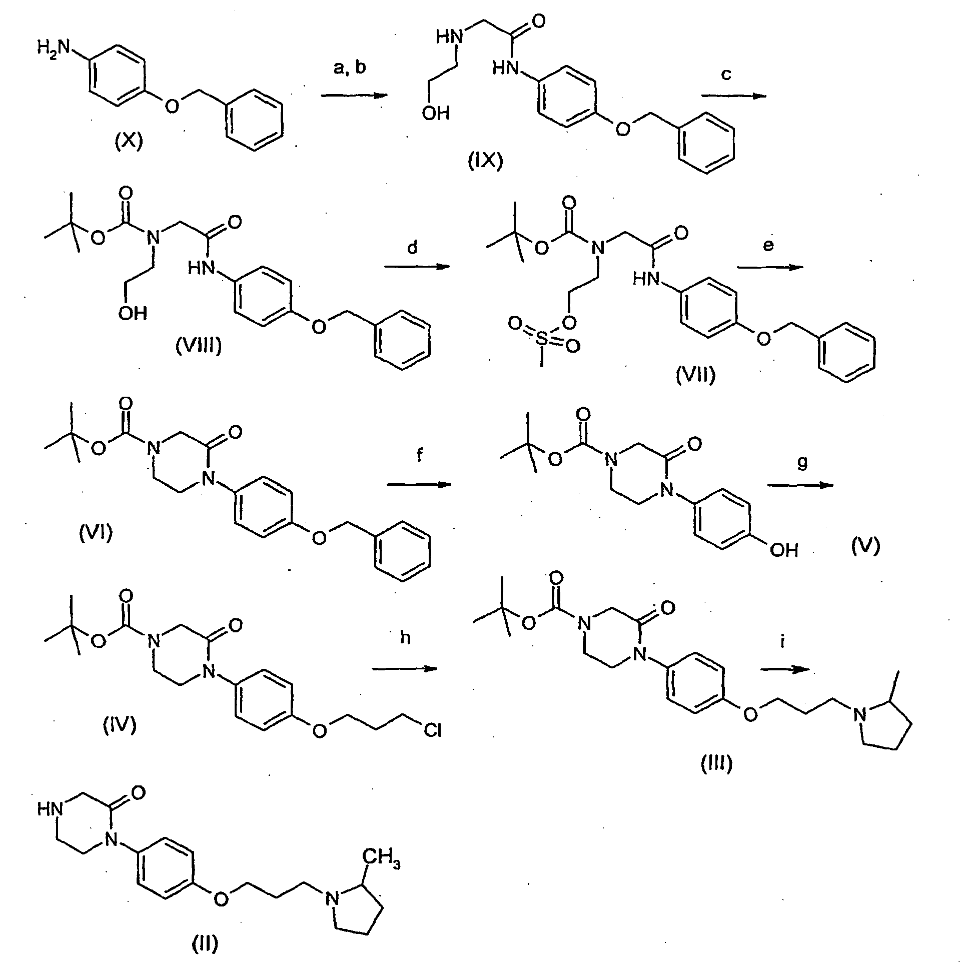

**Reagents and conditions:** a) chloroacetyl chloride, potassium carbonate, tetrahydrofuran; b) ethanolamine, 60 °C; c) Boc₂O, dichloromethane, 4-dimethylaminopyridine; d) methanesulfonyl chloride, triethylamine, dichloromethane; e) NaH, dimethylformamide; f) H₂, 10% palladium on carbon, ethanol; g) 1-bromo-3-chloropropane, potassium carbonate, 2-butanone; h) 2-methylpyrrolidine, 2(R)-methylpyrrolidine or 2(S)-methylpyrrolidine, potasslum carbonate, potassium iodide, 2-butanone; i) trifluoroacetic acid, dichloromethane.

Steps d and e may be performed in one step without the isolation of compound (VII). Alternatively, compound (VII) may be isolated and then subsequently used to prepare compound (VI).

It will be appreciated that other suitable reagents may be substituted for the solvents, bases, protecting groups etc. specifically mentioned above. Further it will be appreciated that reaction conditions such as temperature and reaction times etc. may be varied from the specific conditions described herein, as appropriate, depending on the reagents chosen.

Thus, compound (IX) for example may be protected with benzyloxycarbonyl or 2',2',2'-trichloroethoxycarbonyl groups. The activation of compound (VIII) may be achieved by reaction with tosyl chloride or triflic anhydride in pyridine or in dichloromethane and in the presence of a base such as diisopropylethylamine to provide the corresponding p-toluenesulfonate ester or the trifluoromethanesulfonate ester. Cyclisation of compound (VII) to compound (VI) may be achieved in the presence of other kinetic bases such as sodium bis(trimethylsilyl)amide in tetrahydrofuran. The cleavage of the protecting group from (III) may provide a salt e.g. trifluoroacetic acid salt in the case of clevage of the BOC protecting group with trifluoroacetic acid. The free base can then be isolated following ion exchange chromatography on SCX-2 cartridge. Alternatively, the salt can be used in the acylation reaction in the presence of a base such as triethylamine, diisopropylethylamine or polymer-supported diisopropylethylamine.

According to a second process B, a salt of a compound of formula (I) may be prepared by exchange of counterions, or precipitation of the desired salt from the free base.

Protecting groups that may be employed in the synthetic routes described herein and the means for their removal can be found in T. W. Greene 'Protective Groups in Organic Synthesis' (3rd edition, J. Wiley and Sons, 1999). Suitable amine protecting groups include sulphonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrogen chloride in dioxan or trifluoroacetic acid in dichloromethane) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid.

Further it will be appreciated that the R and S enantiomers may be isolated from the racemate by conventional methods such as preparative HPLC involving a chiral stationary phase, by resolution using fractional crystallisation of a salt of the free base with a chiral acid, by chemical conversion to a diastereoisomer using a chiral auxiliary followed by chromatographic separation of the isomers and then removal of the chiral auxiliary and regeneration of the pure enantiomer, or by total asymmetric synthesis.

It will be appreciated that all novel intermediates used to prepare compounds of the invention form yet another aspect of the present invention.

Examples of disease states in which compounds of formula (I), or pharmaceutically acceptable salts thereof may have potentially beneficial anti-inflammatory and/or anti-allergic effects include diseases of the respiratory tract such as bronchitis (including chronic bronchitis), asthma (including allergen-induced asthmatic reactions), chronic obstructive pulmonary disease (COPD), cystic fibrosis, sinusitis and allergic rhinitis (seasonal and perennial). Other disease states include diseases of the gastrointestinal tract such as intestinal inflammatory diseases including inflammatory bowel disease (e.g. Crohn's disease or ulcerative colitis) and intestinal inflammatory diseases secondary to radiation exposure or allergen exposure.

Furthermore, compounds of the invention may be used to treat nephritis, skin diseases such as psoriasis, eczema, allergic dermatitis and hypersensitivity reactions.

The compounds of the invention may also be of use in the treatment of nasal polyposis, conjunctivitis or pruritis.

Further diseases include inflammatory diseases of the gastrointestinal tract such as inflammatory bowel disease.

A disease of particular interest is allergic rhinitis.

Compounds that are antagonists and/or inverse agonists of the H3 receptor may also be of use in other diseases in which activation of the H3 receptor may be implicated. Such diseases may include non-allergic rhinitis.

It will be appreciated by those skilled in the art that references herein to treatment or therapy extend to prophylaxis as well as the treatment of established conditions.

As mentioned above, compounds of formula (I) are useful as therapeutic agents. There is thus provided, as a further aspect of the invention, a compound of formula (I) or a pharmaceutically acceptable salt thereof for use in therapy.

According to another aspect of the invention, there is provided the use of a compound of formula (I) or a pharmaceutical acceptable salt thereof for the manufacture of a medicament for the treatment of any of the above diseases.

Compounds of formula (I) may be used in a method for the treatment of any of the above diseases, in a human or animal subject in need thereof, which method comprises administering an effective amount of a compound of formula (I) or a pharmaceutical acceptable salt thereof.

When used in therapy, the compounds of formula (I) are usually formulated in a suitable pharmaceutical composition. Such compositions can be prepared using standard procedures.

Thus, the present invention further provides a pharmaceutical composition which comprises a compound of formula (I) or a pharmaceutically acceptable salt thereof optionally with one or more pharmaceutically acceptable carriers and/or excipients.

A composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. A surfactant or wetting agent may be included in the composition to facilitate uniform distribution of the compound.

The composition may contain from about 0.1% to 99% by weight, such as from about 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be about 0.05 to 1000 mg, more suitably about 1.0 to 200 mg, and such unit doses may be administered more than once a day, for example two or three a day. Such therapy may extend for a number of weeks or months. In one embodiment, compounds and compositions according to the invention are suitable for oral administration and/or are capable of once daily administration.

The compounds and compositions according to the invention may be used in combination with or include one or more other therapeutic agents, for example selected from anti-inflammatory agents, anticholinergic agents (particularly an M₁/M₂/M₃ receptor antagonist), β₂-adrenoreceptor agonists, antiinfective agents (e.g. antibiotics, antivirals), or antihistamines. The invention thus provides, in a further aspect, a combination comprising a compound of formula (I) or a pharmaceutically acceptable salt thereof, together with one or more other therapeutically active agents, for example selected from an anti-inflammatory agent (for example another corticosteroid or an NSAID), an anticholinergic agent, a β₂-adrenoreceptor agonist, an antiinfective agent (e.g. an antibiotic or an antiviral), or an antihistamine. Combinations comprising a compound of formula (I) or a pharmaceutically acceptable salt thereof, together with a β₂-adrenoreceptor agonist, and/or an anticholinergic, and/or a PDE-4 inhibitor form yet another aspect of the invention. The combinations of the invention may comprise one or two other therapeutic agents, and may optionally include one or more pharmaceutically acceptable carriers and/or excipients as desired.

It will be clear to a person skilled in the art that, where appropriate, the other therapeutic ingredient(s) may be used in the form of salts, (e.g. as alkali metal or amine salts or as acid addition salts), or prodrugs, or as esters (e.g. lower alkyl esters), or as solvates (e.g. hydrates) to optimise the activity and/or stability and/or physical characteristics (e.g. solubility) of the therapeutic ingredient. It will be clear also that where appropriate, the therapeutic ingredients may be used in optically pure form.

Examples of β₂-adrenoreceptor agonists include salmeterol (e.g. as racemate or a single enantiomer such as the *R*-enantiomer or the S-enantiomer), salbutamol (e.g. as racemate or a single enantiomer such as the *R*-enantiomer), formoterol (e.g. as racemate or a single enantiomer such as the R-enantiomer), salmefamol, fenoterol carmoterol, etanterol, naminterol, clenbuterol, pirbuterol, flerbuterol, reproterol, bambuterol, indacaterol, terbutaline and salts thereof, for example the xinafoate (1-hydroxy-2-naphthalenecarboxylate) salt of salmeterol, the sulphate salt or free base of salbutamol or the fumarate salt of formoterol. Combinations comprising a compound of the invention together with a longer-acting β₂-adrenoreceptor agonist, which provides effective bronchodilation for about 12 hours or longer, may be of particular interest.

Other β₂-adrenoreceptor agonists include those described in WO 02/066422, WO 02/070490, WO 02/076933, WO 03/024439, WO 03/072539, WO 03/091204, WO 04/016578, WO 2004/022547, WO 2004/037807, WO 2004/037773, WO 2004/037768, WO 2004/039762, WO 2004/039766, WO01/42193 and WO03/042160.

Exemplary β₂-adrenoreceptor agonists include:
3-(4-{[6-({(2*R*)-2-hydroxy-2-[4-hydroxy-3-(hydroxymethyl)phenyl]ethyl}amino) hexyl] oxy} butyl) benzenesulfonamide;
3-(3-{[7-({(2*R*)-2-hydroxy-2-[4-hydroxy-3-hydroxymethyl) phenyl] ethyl}-amino) heptyl] oxy} propyl) benzenesulfonamide;
4-{(1*R*)-2-[(6-{2-[(2, 6-dichlorobenzyl) oxy] ethoxy} hexyl) amino]-1-hydroxyethyl}-2-(hydroxymethyl) phenol;
4-{(1*R*)-2-[(6-{4-[3-(cyclopentylsulfonyl)phenyl]butoxy}hexyl)amino]-1-hydroxyethyl}-2-(hydroxymethyl)phenol;
N-[2-hydroxyl-5-[(1*R*)-1-hydroxy-2-[[2-4-[[(2R)-2-hydroxy-2-phenylethyl]amino]phenyl]ethyl]amino]ethyl]phenyl]formamide;
N-2{2-[4-(3-phenyl-4-methoxyphenyl)aminophenyl]ethyl}-2-hydroxy-2-(8-hydroxy-2(1*H*)-quinolinon-5-yl)ethylamine; and
5-[(*R*)-2-(2-{4-[4-(2-amino-2-methyl-propoxy)-phenylamino]-phenyl}-ethylamino)-1-hydroxy-ethyl]-8-hydroxy-1H-quinolin-2-one.

Anti-inflammatory agents include corticosteroids. Corticosteroids which may be used in combination with the compounds of the invention are those oral and inhaled corticosteroids and their pro-drugs which have anti-inflammatory activity. Examples include methyl prednisolone, prednisolone, dexamethasone, fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester, 6α,9α-difluoro-17α-[(2-furanylcarbonyl)oxy]-11β-hydroxy-16α-methyl-3-oxo-androsta-1,4-diene-17β-carbothioic acid S-fluoromethyl ester, 6α,9α-difluoro-11β-hydroxy-16α-methyl-3-oxo-17α-propionyloxy- androsta-1,4-diene-17β-carbothioic acid *S*-(2-oxo-tetrahydro-furan-3S-yl) ester, 6α,9α-difluoro-11β-hydroxy-16α-methyl-3-oxo-17α-(2,2,3,3-tetramethycyclopropylcarbonyl)oxy-androsta-1,4-diene-17β-carbothioic acid S-cyanomethyl ester, 6α,9α-difluoro-11β-hydroxy-16α-methyl-17α-(1-methycyclopropylcarbonyl)oxy-3-oxo-androsta-1,4-diene-17β-carbothioic acid *S-*fluoromethyl ester, beclomethasone esters (eg. the 17-propionate ester or the 17,21-dipropionate ester), budesonide, flunisolide, mometasone esters (eg. the furoate ester), triamcinolone acetonide, rofleponide, ciclesonide (16α,17-[[(*R*)-cyclohexylmethylene]bis(oxy)]-11β,21-dihydroxy-pregna-1,4-diene-3,20-dione), butixocort propionate, RPR-106541, and ST-126. Corticosteroids that may be of interest include fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester and 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester, 6α,9α-difluoro-11β-hydroxy-16α-methyl-3-oxo-17α-(2,2,3,3- tetramethycyclopropylcarbonyl)oxy-androsta-1,4-diene-17β-carbothioic acid *S*-cyanomethyl ester and 6α,9α-difluoro-11β-hydroxy-16α-methyl-17α-(1-methycyclopropylcarbonyl)oxy-3-oxo-androsta-1,4-diene-17β-carbothioic acid S-fluoromethyl ester, especially 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester.

Non-steroidal compounds having glucocorticoid agonism that may possess selectivity for transrepression over transactivation and that may be useful in combination therapy include those covered in the following patents: WO03/082827, WO01/10143, WO98/54159, WO04/005229, WO04/009016, WO04/009017, WO04/018429, WO03/104195, WO03/082787, WO03/082280, WO03/059899, WO03/101932, WO02/02565 WO01/16128, WO00/66590, WO03/086294, WO04/026248, WO03/061651, WO03/08277.

Anti-inflammatory agents include non-steroidal anti-inflammatory drugs (NSAID's). NSAID's include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis (eg. montelukast), iNOS inhibitors, tryptase and elastase inhibitors, beta-2 integrin antagonists and adenosine receptor agonists or antagonists (e.g. adenosine 2a agonists), cytokine antagonists (e.g. chemokine antagonists, such as a CCR3 antagonist) or inhibitors of cytokine synthesis, or 5-lipoxygenase inhibitors. iNOS inhibitors include those disclosed in WO93/13055, WO98/30537, WO02/50021, WO95/34534 and WO99/62875. CCR3 inhibitors include those disclosed in WO02/26722. Adenosine 2a agonists include those disclosed in WO2005/116037.

The PDE4-speciflc inhibitor useful in combinations with compounds of the invention may include any compound that is known to inhibit the PDE4 enzyme or which is discovered to act as a PDE4 inhibitor, and which are only PDE4 inhibitors, not compounds which inhibit other members of the PDE family, such as PDE3 and PDE5, as well as PDE4.

PDE4 inhibitors include *cis*-4-cyano-4-(3-cyclopentyloxy-4-methoxyphenyl)cyclohexan-1-carboxylic acid, 2-carbomethoxy4-cyano-4-(3-cyclopropylmethoxy-4-difluoromethoxyphenyl)cyclohexan-1-one and *cis*-[4-cyano4-(3-cyclopropylmethoxy-4-difluoromethoxyphenyl)cyclohexan-1-ol]. Also, *cis*-4-cyano-4-[3-(cyclopentyloxy)-4-methoxyphenyl]cyclohexane-1-carboxylic acid (also known as cilomilast) and its salts, esters, pro-drugs or physical forms, which is described in U.S. patent 5,552,438 issued 03 September, 1996.

Other PDE4 inhibitors include AWD-12-281 from Elbion (Hofgen, N. et al., 15th EFMC Int. Symp. Med. Chem. (Sept 6-10, Edinburgh) 1998, Abst. P.98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al., Eur. Resp. J. [Ann. Cong. Eur. Resp. Soc. (Sept 19-23, Geneva) 1998] 1998, 12 (Suppl. 28): Abst P2393); roflumilast (CAS reference No 162401-32-3) and a pthalazinone (WO99/47505) from Byk-Gulden; Pumafentrine, (-)-p-[(4*a*R*,10*b*S*)-9-ethoxy-1,2,3,4,4a,10b-hexahydro-8-methoxy-2-methylbenzo[c][1,6]naphthyridin-6-yl]-*N,N-*diisopropylbenzamide which Is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al., J. Pharmacol. Exp. Ther., 284(1):162, 1998), and T2585.

Further compounds of interest are disclosed in the published international patent application WO04/024728 (Glaxo Group Ltd), PCT/EP2003/014867 (Glaxo Group Ltd) and PCT/EP2004/005494 (Glaxo Group Ltd).

Anticholinergic agents are those compounds that act as antagonists at the muscarinic receptors, in particular those compounds which are antagonists of the M₁ or M₃ receptors, dual antagonists of the M₁/M₃ or M₂/M₃, receptors or pan-antagonists of the M₁/M₂/M₃ receptors. Exemplary compounds for administration via inhalation include ipratropium (e.g. as the bromide, CAS 22254-24-6, sold under the name Atrovent), oxitropium (e.g. as the bromide, CAS 30286-75-0) and tiotropium (e.g. as the bromide, CAS 136310-93-5, sold under the name Spiriva). Also of interest are revatropate (e.g. as the hydrobromide, CAS 262586-79-8) and LAS-34273 which is disclosed in WO01/04118. Exemplary compounds for oral administration include pirenzepine (CAS 28797-61-7), darifenacin (CAS 133099-04-4, or CAS 133099-07-7 for the hydrobromide sold under the name Enablex), oxybutynin (CAS 5633-20-5, sold under the name Ditropan), terodiline (CAS 15793-40-5), tolterodine (CAS 124937-51-5, or CAS 124937-52-6 for the tartrate, sold under the name Detrol), otilonium (e.g. as the bromide, CAS 26095-59-0, sold under the name Spasmomen), trospium chloride (CAS 10405-02-4) and solifenacin (CAS 242478-37-1, or CAS 242478-38-2 for the succinate also known as YM-905 and sold under the name Vesicare).

Other anticholinergic agents include compounds of formula (XXI), which are disclosed in US patent application 60/487981: 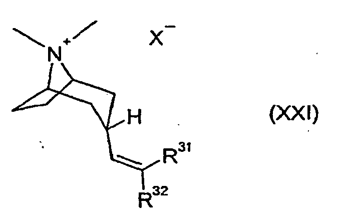 in which the orientation of the alkyl chain attached to the tropane ring may be endo;
R³¹ and R³² are, independently, selected from the group consisting of straight or branched chain lower alkyl groups having, for example, from 1 to 6 carbon atoms, cycloalkyl groups having from 5 to 6 carbon atoms, cycloalkyl-alkyl having 6 to 10 carbon atoms, 2-thienyl, 2-pyridyl, phenyl, phenyl substituted with an alkyl group having not in excess of 4 carbon atoms and phenyl substituted with an alkoxy group having not in excess of 4 carbon atoms;
X⁻ represents an anion associated with the positive charge of the N atom. X⁻ may be but is not limited to chloride, bromide, iodide, sulfate, benzene sulfonate, and toluene sulfonate,
including, for example:
(3-*endo*)-3-(2,2-di-2-thienylethenyl)-8,8-dimethyl-8-azoniabicyclo[3.2.1]octane bromide;
(3-*endo*)-3-(2,2-diphenylethenyl)-8,8-dimethyl-8-azoniabicyclo[3.2.1]octane bromide;
(3-*endo*)-3-(2,2-diphenylethenyl)-8,8-dimethyl-8-azoniabicyclo[3.2.1]octane 4-methylbenzenesulfonate;
(3-*endo*)-8,8-dimethyl-3-[2-phenyl-2-(2-thienyl)ethenyl]-8-azoniabicyclo[3.2.1]octane bromide; and/or
(3-*endo*)-8,8-dimethyl-3-[2-phenyl-2-(2-pyridinyl)ethenyl]-8-azoniabicyclo[3.2.1]octane bromide.

Further anticholinergic agents include compounds of formula (XXII) or (XXIII), which are disclosed in US patent application 60/511009: 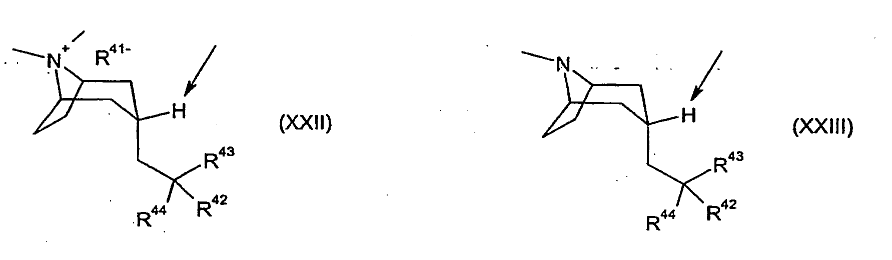 wherein:
the H atom indicated is in the exo position;
R⁴¹ represents an anion associated with the positive charge of the N atom. R⁴¹ may be but is not limited to chloride, bromide, iodide, sulfate, benzene sulfonate and toluene sulfonate;
R⁴² and R⁴³ are independently selected from the group consisting of straight or branched chain lower alkyl groups (having, for example, from 1 to 6 carbon atoms), cycloalkyl groups (having from 5 to 6 carbon atoms), cycloalkyl-alkyl (having 6 to 10 carbon atoms), heterocycloalkyl (having 5 to 6 carbon atoms) and N or O as the heteroatom, heterocycloalkyl-alkyl (having 6 to10 carbon atoms) and N or O as the heteroatom, aryl, optionally substituted aryl, heteroaryl, and optionally substituted heteroaryl;
R⁴⁴ is slected from the group consisting of (C₁-C₆)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocycloalkyl, aryl, heteroaryl, (C₁-C₆)alkyl-aryl, (C₁-C₆)alkyl-heteroaryl, -OR⁴⁵, -CH₂OR⁴⁵, -CH₂OH, -CN, -CF₃, -CH₂O(CO)R⁴⁶, -CO₂R⁴⁷, -CH₂NH₂, -CH₂N(R⁴⁷)SO₂R⁴⁵, -SO₂N(R⁴⁷)(R⁴⁸), -CON(R⁴⁷)(R⁴⁸), -CH₂N(R⁴⁸)CO(R⁴⁶), -CH₂N(R⁴⁸)SO₂(R⁴⁶), -CH₂N(R⁴⁸)CO₂(R⁴⁵), -CH₂N(R⁴⁸)CONH(R⁴⁷);
R⁴⁵ is selected from the group consisting of (C₁-C₆)alkyl, (C₁-C₆)alkyl(C₃-C₁₂)cycloalkyl, (C₁-C₆)alkyl(C₃-C₇)heterocycloalkyl, (C₁-C₆)alkyl-aryl, (C₁-C₆)alkyl-heteroaryl;
R⁴⁶ is selected from the group consisting of (C₁-C₆)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocycloalkyl, aryl, heteroaryl, (C₁-C₆)alkyl-aryl, (C₁-C₆)alkyl-heteroaryl;
R⁴⁷ and R⁴⁸ are, independently, selected from the group consisting of H, (C₁-C₆)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocycloalkyl, (C₁-C₆)alkyl-aryl, and (C₁-C₆)alkyl-heteroaryl, including, for example:
   (Endo)-3-(2-methoxy-2,2-di-thiophen-2-yl-ethyl)-8,8-dimethyl-8-azoniabicyclo[3.2.1]octane iodide;
   3₋((Endo)-8-methyl-8-aza-bicyclo[3.2.1]oct-3-yl)-2,2-diphenyl-propionitrile;
   (Endo)-8-methyl-3-(2,2,2-triphenyl-ethyl)-8-aza-bicyclo[3.2.1]octane;
   3-((Endo)-8-methyl-8-aza-bicyclo[3.2,1]oct-3-yl)-2,2-diphenyl-propionamide;
   3-((Endo)-8-methyl-8-aza-bicyclo[3.2.1]oct-3-yl)-2,2-diphenyl-propionic acid;
   (Endo)-3-(2-cyano-2,2-diphenyl-ethyl)-8,8-dimethyl-8-azonia-bicyclo[3.2.1]octane iodide;
   (Endo)-3-(2-cyano-2,2-diphenyl-ethyl)-8,8-dimethyl-8-azonia-bicyclo[3.2.1]octane bromide;
   3-((Endo)-8-methyl-8-aza-bicyclo[3.2.1]oct-3-yl)-2,2-diphenyl-propan-1-ol;
   *N*-Benzyl-3-((endo)-8-methyl-8-aza-bicyclo[3.2.1]oct-3-yl)-2,2-diphenyl-propionamide;
   (Endo)-3-(2-carbamoyl-2,2-diphenyl-ethyl)-8,8-dimethyl-8-azonia-bicyclo[3.2.1]octane iodide;
   1-Benzyl-3-[3-((endo)-8-methyl-8-aza-bicyclo[3.2.1]oct-3-yl)-2,2-diphenyl-propyl]-urea;
   1-Ethyl-3-[3-((endo)-8-methyl-8-aza-bicyclo[3.2.1]oct-3-yl)-2,2-diphenyl-propyl]-urea;
   *N*-[3-((Endo)-8-methyl-8-aza-bicyclo[3.2.1]oct-3-yl)-2,2-diphenyl-propyl]-acetamide;
   *N*-[3-((Endo)-8-methyl-8-aza-bicyclo[3.2.1]oct-3-yl)-2,2-diphenyl-propyl]-benzamide;
   3-((Endo)-8-methyl-8-aza-bicyclo[3.2.1]oct-3-yl)-2,2-di-thiophen-2-yl-propionitrile;
   (Endo)-3-(2-cyano-2,2-di-thiophen-2-yl-ethyl)-8,8-dimethyl-8-azonia-bicyclo[3.2.1]octane iodide;
   *N*-[3-((Endo)-8-methyl-8-aza-bicyclo[3.2.1]oct-3-yl)-2,2-diphenyl-propyl]-benzenesulfonamide;
   [3-((Endo)-8-methyl-8-aza-bicyclo[3.2.1]oct-3-yl)-2,2-diphenyl-propyl]-urea;
   *N*-[3-((Endo)-8-methyl-8-aza-bicyclo[3.2.1]oct-3-yl)-2,2-diphenyl-propyl]-methanesulfonamide; and/or
   (Endo)-3-{2,2-diphenyl-3-[(1-phenyl-methanoyl)-amino]-propyl}-8,8-dimethyl-8-azoniabicyclo[3.2.1]octane bromide.
Compounds of particular interest that may be useful in combinations of the invention include:
(Endo)-3-(2-methoxy-2,2-di-thiophen-2-yl-ethyl)-8,8-dimethyl-8-azonia-bicyclo[3.2.1]octane iodide;
(Endo)-3-(2-cyano-2,2-diphenyl-ethyl)-8,8-dimethyl-8-azonia-bicyclo[3.2.1]octane iodide;
(Endo)-3-(2-cyano-2,2-diphenyl-ethyl)-8,8-dimethyl-8-azonia-bicyclo[3.2.1]octane bromide;
(Endo)-3-(2-carbamoyl-2,2-diphenyl-ethyl)-8,8-dimethyl-8-azonia-bicyclo[3.2.1]octane iodide;
(Endo)-3-(2-cyano-2,2-di-thiophen-2-yl-ethyl)-8,8-dimethyl-8-azonia-bicyclo[3.2.1]octane iodide; and/or
(Endo)-3-{2,2-diphenyl-3-[(1-phenyl-methanoyl)-amino]-propyl}-8,8-dimethyl-8-azoniabicyclo[3.2.1]octane bromide.

Of particular interest is a combination comprising a compound of formula (I) or a pharmaceutically acceptable salt thereof together with an H1 antagonist. Suitable H1 antagonists include, without limitation, amelexanox, astemizole, azatadine, azelastine, acrivastine, brompheniramine, cetirizine, levocetirizine, efletirizine, chlorpheniramine, clemastine, cyclizine, carebastine, cyproheptadine, carbinoxamine, descarboethoxyloratadine, doxylamine, dimethindene, ebastine, epinastine, efletirizine, fexofenadine, hydroxyzine, ketotifen, loratadine, levocabastine, mizolastine, mequitazine, mianserin, noberastine, meclizine, norastemizole, olopatadine, picumast, pyrilamine, promethazine, terfenadine, tripelennamine, temelastine, trimeprazine and triprolidine, particularly cetirizine, levocetirizine, efletirizine and fexofenadine. Other histamine receptor antagonists which may be used alone, or in combination with an H3 receptor antagonist include antagonists (and/or inverse agonists) of the H4 receptor, for example, the compounds disclosed in Jablonowski et al., J. Med. Chem. 46:3957-3960 (2003).

The invention thus provides, in a further aspect, a combination comprising a compound of formula (I) or a pharmaceutically acceptable salt thereof, together with a PDE4 inhibitor.

The invention thus provides, in a further aspect, a combination comprising a compound of formula (I) or a pharmaceutically acceptable salt thereof, together with a β₂-adrenoreceptor agonist.

The invention thus provides, in a further aspect, a combination comprising a compound of formula (I) or a pharmaceutically acceptable salt thereof, together with an anticholinergic.

The invention thus provides, in a further aspect, a combination comprising a compound of formula (I) or a pharmaceutically acceptable salt thereof, together with a H1 receptor antagonist.

The invention thus provides, in a further aspect, a combination comprising a compound of formula (I) or a pharmaceutically acceptable salt thereof, together with a corticosteroid.

The invention thus provides, in a further aspect, a combination comprising a compound of formula (I) or a pharmaceutically acceptable salt thereof, together with a A2a receptor agonist.

The combinations referred to above may conveniently be presented for use in the form of a composition and thus compositions comprising a combination as defined above, optionally together with a pharmaceutically acceptable diluent, carrier or excipients represent a further aspect of the invention.

The individual compounds of such combinations may be administered either sequentially or simultaneously in separate or combined compositions. Suitably, the individual compounds will be administered simultaneously in a combined composition. Appropriate doses of known therapeutic agents will be readily appreciated by those skilled in the art.

It will be clear to a person skilled in the art that, where appropriate, the other therapeutic ingredient(s) may be used in the form of salts, for example as alkali metal or amine salts or as acid addition salts, or prodrugs, or as esters, for example lower alkyl esters, or as solvates, for example hydrates, to optimise the activity and/or stability and/or physical characteristics, such as solubility, of the therapeutic ingredient. It will be clear also that, where appropriate, the therapeutic ingredients may be used in optically pure form.

The compounds of the invention may be prepared by the methods described below or by similar methods. Thus, the following Descriptions and Examples illustrate the preparation of compounds of the invention. The Examples are not to be considered as limiting the scope of the invention in any way.

### GENERAL EXPERIMENTAL

Throughout the examples, the following abbreviations are used:
DCM: dichloromethane
DMF: *N,N*-dimethylformamide
EtOAc: ethylacetate
h: hour(s)
LCMS: Liquid Chromatography Mass Spectrometry
min: minute(s)
MgSO₄: magnesium sulfate
PS-DIPEA: polymer supported diisopropylethylamine
RT: retention time
TBTU: *O*-(benzotriazol-1-yl)-*N,N,N,N'*-tetramethyluronium tetrafluoroborate

SCX cartridges are Ion Exchange SPE columns where the stationary phase is polymeric benzene sulfonic acid. These are used to isolate amines.
SCX2 cartridges are Ion Exchange SPE columns where the stationary phase is polymeric propylsulfonic acid. These are used to isolate amines.

Organic solutions were dried either over magnesium or sodium sulfate.

LCMS was conducted on a Supelcosil LCABZ+PLUS column (3.3 cm × 4.6 mm ID) eluting with 0.1% formic acid and 0.01 M ammonium acetate in water (solvent A) and 0.05% formic acid 5% water in acetonitrile (solvent B), using the following elution gradient 0.0 - 7min 0% B, 0.7 - 4.2 min 100% B, 4.2 - 5.3 min 0% B, 5.3 - 5.5min 0% B at a flow rate of 3 mlmin⁻¹. The mass spectra were recorded on a Fisons VG Platform spectrometer using electrospray positive and negative mode (ES+ve and ES-ve).

The Flashmaster II is an automated multi-user flash chromatography system, available from Argonaut Technologies Ltd, which utilises disposable, normal phase, SPE cartridges (2 g to 100 g). It provides quaternary on-line solvent mixing to enable gradient methods to be run. Samples are queued using the multi-functional open access software, which manages solvents, flow-rates, gradient profile and collection conditions. The system is equipped with a Knauer variable wavelength UV-detector and two Gilson FC204 fraction-collectors enabling automated peak cutting, collection and tracking.

Mass directed autopreparative (MDAP) HPLC was conducted on a Waters FractionLynx system comprising of a Waters 600 pump with extended pump heads, Waters 2700 autosampler, Waters 996 diode array and Gilson 202 fraction collector on a 10 cm × 2.54 cm id ABZ+ column, eluting with 0.1 % formic acid in water (solvent A) and 0.1 % formic acid in acetonitrile (solvent B), using as appropriate elution gradient over 15 min at a flow rate of 20 mlmin⁻¹ and detecting at 200 - 320 nm at room temperature. Mass spectra were recorded on Micromass ZMD mass spectrometer using electro spray positive and negative mode, alternate scans. The software used was *MassLynx* 3.5 with *OpenLynx* and *FractionLynx* options.

Compounds were named using ACD/Name PRO 6.02 chemical naming software Advanced Chemistry Developments Inc.; Toronto, Ontario, M5H2L3, Canada.

### Intermediate 1

### N²-(2-Hydroxyethyl)-N¹-{4-[(phenylmethyl)oxy]phenyl}glycinamide

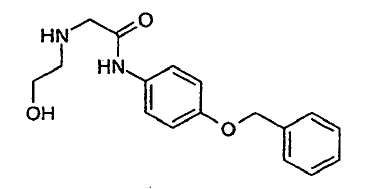

4-[(Phenylmethyl)oxy]aniline hydrochloride salt (available from Fisher for example) (10 g) was stirred in dry tetrahydrofuran (100 ml), and cooled in an ice/water bath to approximately 5 °C. A solution of potassium carbonate (16.15 g) in water (60 ml) was added to the above mixture, followed by the dropwise addition of chloroacetyl chloride (4.22 ml) over 30 min. The mixture was allowed to warm up to room temperature and the organic phase separated. The organic phase was cooled to approximately 5°C in an ice/water bath and 2-aminoethanol (9 g) was added. The mixture was allowed to warm up to room temperature and then heated at 60 °C for 2 h, left overnight at room temperature and heated at 60 °C for a further 2 h. The reaction mixture was partitioned between ethyl acetate and water. The organic phase was separated, washed with water, brine, dried (MgSO₄) and concentrated. The residue was recrystallised from chloroform to give *the title compound* (8.1 g). LCMS RT = 2.17 min, ES+ve *m*/*z* 301 (M+H)⁺.

### Intermediate 2

### 1,1-Dimethylethyl (2-hydroxyethyl)[2-oxo-2-({4-[(phenylmethyl)oxy]phenyl}amino)ethyl]carbamate

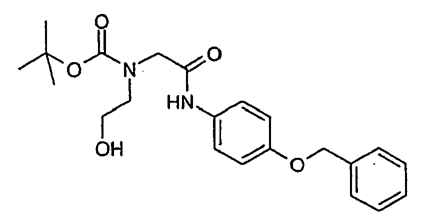

Bis(1,1-dimethylethyl) dicarbonate (available from Fisher for example) (14.5 g) and 4-dimethylaminopyridine (available from Aldrich for example) (150 mg) were added to a solution of *N*²-(2-hydroxyethyl)-*N*¹-{4-[(phenylmethyl)oxy]phenyl}glycinamide (Intermediate 1) (8.1 g) in DCM (200 ml). The mixture was stirred for 30 min, and aqueous saturated sodium bicarbonate added (150 ml). The organic phase was separated, dried (MgSO₄) and evaporated. The residue was dissolved in methanol (150 ml) and potassium carbonate (14.5 g) added. The mixture was heated on a steam bath for 15 min, allowed to cool to room-temperature and left standing for 18 h. The reaction mixture was evaporated and partitioned between DCM and water. The organic phase was separated, washed with brine, dried (MgSO₄) and evaporated. The residue was pre-absorbed onto flash silica and purified by flash chromatography, (100 g silica cartridge; 0% to 100% EtOAc - cyclohexane gradient over 30 min), to give *the title compound* (5.0 g). LCMS RT = 3.2 min, ES+ve *m*/*z* 401 (M+H)⁺.

### Intermediate 3

### 1,1-Dimethylethyl 3-oxo-4-{4-[(phenylmethyl)oxy]phenyl}-1-piperazinecarboxylate

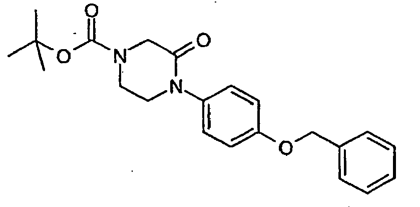

Methanesulfonyl chloride (2.5 ml) was added to a solution of 1,1-dimethylethyl (2-hydroxyethyl)[2-oxo-2-({4-[(phenylmethyl)oxy]phenyl}amino)ethyl]carbamate (Intermediate 2) (4.99 g) in dry DCM (50 ml) and triethylamine (20 ml) under nitrogen. The mixture was stirred for 30 min, and then partitioned between DCM and aqueous saturated sodium bicarbonate. The organic layer was separated and concentrated *in vacuo*. The residue was dissolved in dry DMF (20 ml) and sodium hydride (60% oil dispersion, 0.6 g) added. The mixture was stirred for 2.5 h, quenched with aqueous saturated sodium bicarbonate and extracted with EtOAc × 3. The organic phases were combined, dried (MgSO₄) and evaporated. The residue was purified by flash chromatography, (100 g silica cartridge; 0% to 100% EtOAc - cyclohexane gradient over 40 min), to give *the title compound* (4.1 g). LCMS RT = 3.2 min, ES+ve *m*/*z* 383 (M+H)⁺.

### Intermediate 4

### 1,1-Dimethylethyl 4-(4-hydroxyphenyl)-3-oxo-1-piperazinecarboxylate

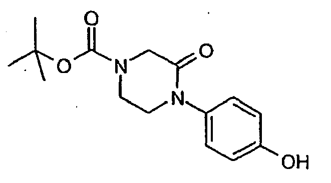

A solution of 1,1-dimethylethyl 3-oxo-4-{4-[(phenylmethyl)oxy]phenyl}-1-piperazinecarboxylate (Intermediate 3) (3.1 g) in ethanol (25 ml) and EtOAc (25 ml) was hydrogenated over 10% palladium on carbon (1 g) for 2 h. The catalyst was removed by filtration through celite and washed with ethanol. The combined filtrate and washings were concentrated to give *the title compound* (1.92 g). LCMS RT = 2.5 min, ES+ve *mlz* 293 (M+H)⁺.

### Intermediate 5

### 1,1-Dimethylethyl 4-{4-[(3-chloropropyl)oxy]phenyl}-3-oxo-1-piperazinecarboxylate

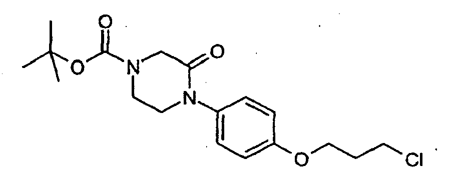

To a solution of 1,1-dimethylethyl 4-(4-hydroxyphenyl)-3-oxo-1-piperazinecarboxylate (Intermediate 4) (5.0 g) in 2-butanone (200 ml) was added potassium carbonate (4.7 g) followed by 1-bromo-3-chloropropane (4.0 g). The mixture was heated at reflux under nitrogen over the weekend (for about 72 h), cooled to room temperature and water (200 ml) added. The organic phase was separated, and the aqueous layer extracted twice with DCM. The organic phases were combined, dried (MgSO₄) and evaporated. The residue was purified by flash chromatography, (100 g silica cartridge; 0% to 100% EtOAc - cyclohexane gradient over 60 min), to give a mixture of *the title compound* and 1,1-dimethylethyl 4-{4-[(3-bromopropyl)oxy]phenyl}-3-oxo-1-piperazinecarboxylate (6.2 g; 9:1). LCMS RT = 3.2 min, ES+ve *m*/*z* 369/371 (3:1) (M⁺H)⁺ for the chloride and RT = 3.26 min, ES+ve *m*/*z* 413/415 (1:1) (M+H)⁺ for the bromide.

### Intermediate 6

### 1,1-Dimethylethyl 4-[4-({3-[(2R)-2-methyl-1-pyrrolidinyl]propyl}oxy)phenyl]-3-oxo-1-piperazinecarboxylate

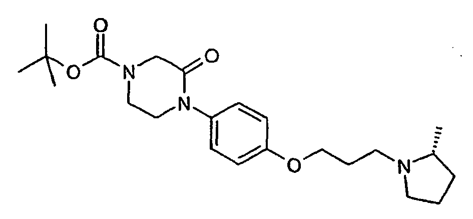

A mixture of 1,1-dimethylethyl 4-{4-[(3-chloropropyl)oxy]phenyl}-3-oxo-1-piperazinecarboxylate and 1,1-dimethylethyl 4-{4-[(3-bromopropyl)oxy]phenyl}-3-oxo-1-piperazinecarboxylate (Intermediate 5) (9:1; 2.58 g) was dissolved in 2-butanone (50 ml). Potassium carbonate (2.9 g), potassium iodide (2.32 g) and (2*R*)-2-methylpyrrolidine hydrochloride (which can be made as described in US 20040171845 for example)(1.0 g) were added. The mixture was heated at reflux under nitrogen over the weekend (for about 72 h). The reaction mixture was cooled to room temperature and partitioned between DCM and water. The organic phase was dried (MgSO₄) and concentrated. The residue was purified by flash chromatography, [100 g silica cartridge; 0% to 30% of 1% triethylamine in methanol - DCM gradient over 60 min], to give *the title compound* (1.87 g). LCMS RT = 2.2 min, ES+ve *m*/*z* 418 (M+H)⁺.

### Intermediate 7

### 1-[4-({3-[(2R)-2-Methyl-1-pyrrolidinyl]propyl}oxy)phenyl]-2-piperazinone

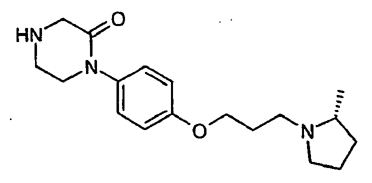

A solution of trifluoroacetic acid (4 ml) in DCM (6 ml) was added to 1,1-dimethylethyl 4-[4-({3-[(2*R*)-2-methyl-1-pyrrolidinyl]propyl}oxy)phenyl]-3-oxo-1-piperazinecarboxylate (Intermediate 6) (1.87 g) in DCM (10 ml). The mixture was stirred under nitrogen for 2 h. Trifluoroacetic acid (4 ml) was added and the mixture stirred for a further 18 h. The mixture was evaporated and the residue dissolved in methanol. The solution was applied to an SCX-2 cartridge (70 g). The cartridge was washed with methanol and the product eluted with 10% 0.880 ammonia in methanol. The appropriate ammoniacal fractions were combined and concentrated to give the *title compound* (1.39 g). LCMS RT = 0.35 min, ES+ve *m*/*z* 318 (M+H)⁺.

### Intermediate 8

### 1,1-Dimethylethyl 4-(4-{[3-(2-methyl-1-pyrrolidinyl)propyl]oxy}phenyl)-3-oxo-1-piperazinecarboxylate

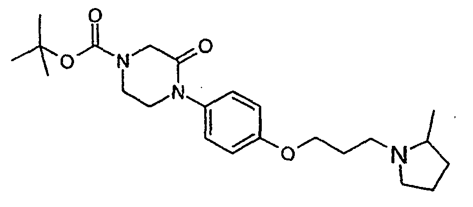

A solution of 1,1-dimethylethyl 4-{4-[(3-chloropropyl)oxy]phenyl}-3-oxo-1-piperazinecarboxylate (Intermediate 5) (1.3 g) was dissolved in 2-butanone (50 ml). Potassium carbonate (0.96 g), potassium iodide (1.16 g) and 2-methylpyrrolidine (available from Fisher for example)(1.5 g) were added. The mixture was heated at 80 °C for 24 h. The reaction mixture was cooled to room temperature and partitioned between EtOAc (x 2) and water. The organic phases were combined and concentrated. The residue was purified by Flashmaster II, [100 g silica cartridge; 0% to 15% (10% 0.880 ammonia in methanol) - DCM gradient over 30 min]. The appropriate fractions were combined and concentrated to give the *title compound* (0.85 g). LCMS RT = 2.28 min, ES+ve *m*/*z* 418 (M+H)⁺.

### Intermediate 9

### 1-(4-{[3-(2-Methyl-1-pyrrolidinyl)propyl]oxy}phenyl)-2-piperazinone

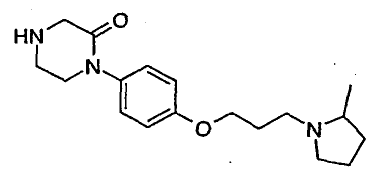

1,1-Dimethylethyl 4-[4-({3-[(2-methyl-1-pyrrolidinyl]propyl}oxy)phenyl]-3-oxo-1-piperazinecarboxylate (Intermediate 8) (0.85 g) was dissolved in DCM (10 ml) and trifluoroacetic acid - DCM mixture (10 ml, 2:5) added. The reaction mixture was stirred at room temperature for 18 h. The solvent was removed *in vacuo*. The residue was dissolved in methanol and the solution was applied to an SCX-2 cartridge (70 g). The cartridge was washed with methanol and the product eluted with 10% 0.880 ammonia in methanol. The appropriate ammoniacal fractions were combined and concentrated to give the *title compound* (645 mg). LCMS RT = 0.36 min, ES+ve *m*/*z* 318 (M+H)⁺.

### Intermediate 10

### 1,1-Dimethylethyl 4-[4-({3-[(2S)-2-methyl-1-pyrrolidinyl]propyl}oxy)phenyl]-3-oxo-1-piperazinecarboxylate

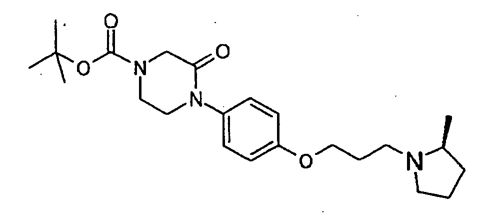

A solution of 1,1-dimethylethyl 4-{4-[(3-chloropropyl)oxy]phenyl}-3-oxo-1-piperazinecarboxylate (Intermediate 5) (0.74 g) was dissolved in 2-butanone (25 ml). Potassium carbonate (0.55 g), potassium iodide (0.66 g) and (2*S*)-2-methylpyrrolidine hydrochloride (which can be made by the methods described in US20040171845 for example)(0.2 g) were added. The mixture was heated at 80 °C overnight (about 18 hours). The reaction mixture was cooled to room temperature and partitioned between DCM and water. The organic phase was dried and concentrated. The residue was purified first by FLashmaster II chromatography, [70 g silica cartridge; 0% to 30% (1% triethylamine in methanol) - DCM gradient over 40 min]. The appropriate ammoniacal fractions were combined and concentrated. The residue was taken up in MeOH and applied to a 20 g SCX-2 ion exchange cartridge and eluted with 10% 0.880 ammonia in methanol solution to give the *title compound* (0.418 g). LCMS RT = 2.2 min, ES+ve *m*/*z* 418 (M+H)⁺.

### Intermediate 11

### 1-[4-({3-1(2S)-2-Methyl-1-pyrrolidinyl]propyl}oxy)phenyl]-2-piperazinone

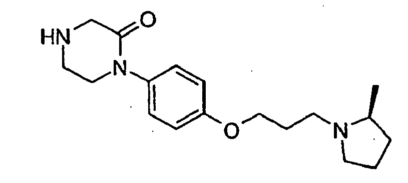

1,1-Dimethylethyl 4-[4-({3-[(2*S*)-2-methy)-1-pyrrolidinyl]propyl}oxy)phenyl]-3-oxo-1-piperazinecarboxylate (Intermediate 10) (0.418 g) was dissolved in DCM (10 ml) and trifluoroacetic acid (2 ml) added. The reaction mixture was stirred at room temperature for 2 to 3 h. The solvent was removed *in vacuo* to leave *the title compound* (0.32 g). LCMS RT = 0.37 min, ES+ve *m*/*z* 318 (M+H)⁺.

### Example 1

### 4-[(2,4-Difluorophenyl)carbonyl]-1-[4-({3-[(2R)-2-methyl-1-pyrrolidinyl]propyl}oxy)phenyl]-2-piperazinone, formate salt

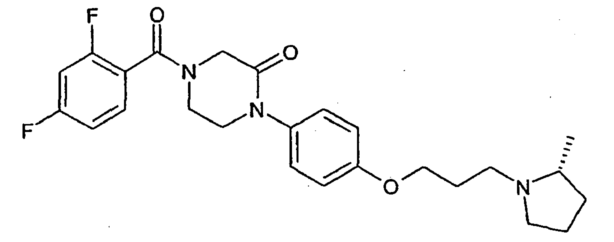

A solution of 1-[4-({3-[(2*R*)-2-methyl-1-pyrrolidinyl]propyl}oxy)phenyl]-2-piperazinone (Intermediate 7) (159 mg) in dry DMF (1ml) and triethylamine (0.21 ml) was added to a mixture of 2,4-difluorobenzoic acid (79 mg) and TBTU (0.21 g) in dry DMF (2 ml). The solution was stirred at room temperature for 18 h, and then diluted with methanol. This solution was applied to an SCX-2 cartridge (20 g). The cartridge was washed with methanol and the product eluted with 10% 0.880 ammonia in methanol. The appropriate ammoniacal fractions were combined and concentrated. The residue was purified by mass-directed auto-preparative HPLC to give *the title compound* (197 mg). LCMS RT = 2.14 min, ES+ve *m*/*z* 458 (M+H)⁺. ¹H NMR δ (CD₃OD) 8.53 (1H, s), 7.56 (1H, m), 7.25 (2H, m), 7.13 (2H, m), 6.99 (2H, m), 4.48 (55% 2H, s), 4.18 - 4.07 [(45% 2H) + 4H + (55% 1H), t + m)], 3.85 - 2.95 (7H + 45% 1H, m), 2.30 - 2.10 (3H, m), 2.08 - 1.98 (2H, m), 1.75 - 1.62 (1H, m), 1.38 (3H, d).

### Example 2

### 4-[(2,4-Difluorophenyl)carbonyl]-1-[4-({3-[2-methyl-1-pyrrolidinyl]propyl}oxy)phenyl]-2-piperazinone, formate salt

Compound was prepared in an analogous method to that described for Example 1, using Intermediate 9 and 2,4-difluorobenzoic acid to give *the title compound* LCMS RT = 2.03 min, ES+ve *m*/*z* 458 (M+H)⁺.

### Example 3

### 4-[(2,4-Difluorophenyl)carbonyl]-1-[4-({3-[(2S)-2-methyl-1-pyrrolidinyl]propyl}oxy)phenyl]-2-piperazinone, formate salt

Compound was prepared in an analogous method to that described for Example 1 using Intermediate 11 and 2,4-difluorobenzoic acid to give *the title compound* LCMS RT = 2.14 min, ES+ve *m*/*z* 458 (M+H)⁺.

### Biological Data

Compounds of the invention may be tested for *in vitro* biological activity in accordance with the following or similar assays:

### H1 receptor cell line generation and FLIPR assay protocol

### 1. Generation of histamine H1 cell line

The human H1 receptor was cloned using known procedures described in the literature [Biochem. Biophys. Res. Commun., 201(2):894, (1994)]. Chinese hamster ovary cells stably expressing the human H1 receptor were generated according to known procedures described in the literature [Br. J. Pharmacol., 117(6):1071, (1996)].

### Histamine H1 functional antagonist assay

The histamine H1 cell line was seeded into non-coated black-walled clear bottom 384-well tissue culture plates in alpha minimum essential medium (Gibco/Invitrogen, cat. no. 22561-021), supplemented with 10% dialysed foetal calf serum (Gibco/Invitrogen cat. no. 12480-021) and 2 mM L-glutamine (Gibco/Invitrogen cat. No. 25030-024) and maintained overnight at 5% CO₂, 37 °C.

Excess medium was removed from each well to leave 10 µl. 30 µl loading dye (250 µM Brilliant Black, 2 µM Fluo-4 diluted in Tyrodes buffer + probenecid (145 mM NaCl, 2.5 mM KCI, 10 mM HEPES, 10 mM D-glucose, 1.2 mM MgCl₂, 1.5 mM CaCl₂, 2.5 mM probenecid, pH adjusted to 7.40 with NaOH 1.0 M)) was added to each well and the plates were incubated for 60 min at 5% CO₂, 37 °C.

10 µl of test compound, diluted to the required concentration in Tyrodes buffer + probenecid (or 10 µl Tyrodes buffer + probenecid as a control) was added to each well and the plate incubated for 30 min at 37 °C, 5% CO_{2.} The plates were then placed into a FLIPR™ (Molecular Devices, UK) to monitor cell fluorescence (λₑₓ = 488 nm, λ_{EM} = 540 nm) in the manner described in Sullivan et al. (In: Lambert DG (ed.), Calcium Signaling Protocols, New Jersey: Humana Press, 1999, pp. 125-136) before and after the addition of 10 µl histamine at a concentration that results in the final assay concentration of histamine being EC₈₀.

Functional antagonism is indicated by a suppression of histamine induced increase in fluorescence, as measured by the FLIPR™ system (Molecular Devices). By means of concentration effect curves, functional affinities are determined using standard pharmacological mathematical analysis.

### 2. H3 receptor cell line generation, membrane preparation and functional GTPγS assay protocols

### Generation of histamine H3 cell line

The histamine H3 cDNA was isolated from its holding vector, pCDNA3.1 TOPO (InVitrogen), by restriction digestion of plasmid DNA with the enzymes BamH1 and Not-1 and ligated into the inducible expression vector pGene (InVitrogen) digested with the same enzymes. The GeneSwitch™ system (a system where in transgene expression is switched off in the absence of an inducer and switched on in the presence of an inducer) was performed as described in US Patent nos: 5,364,791; 5,874,534; and 5,935,934. Ligated DNA was transformed into competent DH5α *E*. *coli* host bacterial cells and plated onto Luria Broth (LB) agar containing Zeocin™ (an antibiotic which allows the selection of cells expressing the *sh ble* gene which is present on pGene and pSwitch) at 50 µgml⁻¹. Colonies containing the re-ligated plasmid were identified by restriction analysis. DNA for transfection into mammalian cells was prepared from 250 ml cultures of the host bacterium containing the pGeneH3 plasmid and isolated using a DNA preparation kit (Qiagen Midi-Prep) as per manufacturers guidelines (Qiagen).

CHO K1 cells previously transfected with the pSwitch regulatory plasmid (InVitrogen) were seeded at 2×10⁶ cells per T75 flask in Complete Medium, containing Hams F12 (GIBCOBRL, Life Technologies) medium supplemented with 10% v/v dialysed foetal bovine serum, L-glutamine, and hygromycin (100 µgml⁻¹), 24 hours prior to use. Plasmid DNA was transfected into the cells using Lipofectamine plus according to the manufacturers guidelines (InVitrogen). 48 hours post transfection cells were placed into complete medium supplemented with 500 µgml⁻¹ Zeocin™.

10-14 days post selection 10 nM Mifepristone (InVitrogen), was added to the culture medium to induce the expression of the receptor. 18 hours post induction cells were detached from the flask using ethylenediamine tetra-acetic acid (EDTA; 1:5000; InVitrogen), following several washes with phosphate buffered saline pH 7.4 and resuspended in Sorting Medium containing Minimum Essential Medium (MEM), without phenol red, and supplemented with Earles salts and 3% Foetal Clone II (Hyclone). Approximately 1×10⁷ cells were examined for receptor expression by staining with a rabbit polyclonal antibody, 4a, raised against the *N*-terminal domain of the histamine H3 receptor, incubated on ice for 60 min, followed by two washes in sorting medium. Receptor bound antibody was detected by incubation of the cells for 60 min on ice with a goat anti rabbit antibody, conjugated with Alexa 488 fluorescence marker (Molecular Probes). Following two further washes with Sorting Medium, cells were filtered through a 50 µm Filcon™ (BD Biosciences) and then analysed on a FACS Vantage SE Flow Cytometer fitted with an Automatic Cell Deposition Unit. Control cells were non-induced cells treated in a similar manner. Positively stained cells were sorted as single cells into 96-well plates, containing Complete Medium containing 500 µgml⁻¹ Zeocin™ and allowed to expand before reanalysis for receptor expression via antibody and ligand binding studies. One clone, 3H3, was selected for membrane preparation.

### Membrane preparation from cultured cells

All steps of the protocol are carried out at 4 °C and with pre-cooled reagents. The cell pellet is resuspended in 10 volumes of homogenisation buffer (50 mM *N*-2-hydroxyethylpiperazine-*N*-2-ethanesulfonic acid (HEPES), 1 mM ethylenediamine tetra-acetic acid (EDTA), pH 7.4 with KOH, supplemented with 10⁻⁶ M leupeptin (acetyl-leucyl-leucyl-arginal; Sigma L2884), 25 µgml⁻¹ bacitracin (Sigma B0125), 1 mM phenylmethylsulfonyl fluoride (PMSF) and 2×10⁻⁶ M pepstain A (Sigma)). The cells are then homogenised by 2 × 15 second bursts in a 1 litre glass Waring blender, followed by centrifugation at 500 g for 20 min. The supernatant is then spun at 48,000 g for 30 min. The pellet is resuspended in homogenisation buffer (4x the volume of the original cell pellet) by vortexing for 5 seconds, followed by homogenisation in a Dounce homogeniser (10 - 15 strokes). At this point the preparation is aliquoted into polypropylene tubes and stored at -80°C.

### Histamine H3 functional antagonist assay

For each compound being assayed, in a solid white 384 well plate, is added:-
(a) 0.5 µl of test compound diluted to the required concentration in DMSO (or 0.5 µl DMSO as a control);
(b) 30 µl bead/membrane/GDP mix prepared by mixing Wheat Germ Agglutinin Polystyrene LeadSeeker® (WGA PS LS) scintillation proximity assay (SPA) beads with membrane (prepared in accordance with the methodology described above) and diluting in assay buffer (20 mM *N*-2-Hydroxyethylpiperazine-*N'*-2-ethanesulfonic acid (HEPES) + 100 mM NaCl + 10 mM MgCl₂, pH 7.4 NaOH) to give a final volume of 30 µl which contains 5 µg protein and 0.25 mg bead per well, incubating at room temperature for 60 min on a roller and, just prior to addition to the plate, adding 10 µM final concentration of guanosine 5'diphosphate (GDP) (Sigma; diluted in assay buffer);
(c) 15 µl 0.38 nM [³⁵S]-GTPγS (Amersham; Radioactivity concentration = 37 MBqml⁻¹; Specific activity = 1160 Cimmol⁻¹), histamine (at a concentration that results in the final assay concentration of histamine being EC₈₀).

After 2-6 hours, the plate is centrifuged for 5 min at 1500 rpm and counted on a Viewlux counter using a 613/55 filter for 5 minplate⁻¹. Data is analysed using a 4-parameter logistical equation. Basal activity used as minimum i.e. histamine not added to well.

Bioavailability and CNS penetration of compounds of the invention may be assayed in the following, or similar, assays.

### 1. CNS Penetration Method

Compounds were dosed intravenously at a nominal dose level of 1 mgkg⁻¹ to male CD Sprague Dawley rats. Compounds were formulated in 5% DMSO/45% PEG200/50% water. Blood samples were taken under terminal anaesthesia with isoflurane at 5 min post-dose and the brains were also removed for assessment of brain penetration. Blood samples were taken directly into heparinised tubes. Blood samples were prepared for analysis using protein precipitation and brain samples were prepared using extraction of drug from brain by homogenisation and subsequent protein precipitation. The concentration of parent drug in blood and brain extracts was determined by quantitative LC-MS/MS analysis using compound-specific mass transitions.

### 2. Rat Pharmacokinetics Method

Compounds were dosed to male CD Sprague Dawley rats by single intravenous or oral administration at a nominal dose level of 1 mgkg⁻¹ and 3 mgkg⁻¹ respectively. Compounds were formulated in 5% DMSO/45% PEG200/50% water. An intravenous profile was obtained by taking serial or terminal blood samples at 0.083, 0.25, 0.5, 1, 2, 4, and 7 h post-dose. An oral profile was obtained by taking serial or terminal blood samples at 0.25, 0.5, 1, 2, 4, 7 and 12 h post-dose. Blood samples were taken directly into heparinised tubes. Blood samples were prepared by protein precipitation and subjected to quantitative analysis by LC-MS/MS using compound-specific mass transitions. Drug concentration-time profiles were generated and non-compartmental PK analysis used to generate estimates of half-life, clearance, volume of distribution and oral bioavailability.

### 3. Dog Pharmacokinetics Method

Compounds were dosed to male Beagle dogs by single intravenous or oal administration at a nominal dose level of 1 mgkg⁻¹ and 2 mgkg⁻¹ respectively. The study was carried out according to a crossover design such that the same dog was used for both dosing events and the dosing events occurred 1 week apart. Compounds were formulated in 5%DMSO/45%Peg200/50%water. An intravenous profile was obtained by taking serial blood samples at 0.083, 0.25, 0.5, 0.75, 1, 2, 4, 6 & 12 h post-dose. An oral profile was obtained by taking serial blood samples at 0.25, 0.5, 0.75, 1, 2, 4, 6, 12 & 24 h post-dose. Blood samples were taken directly into heparinised tubes. Blood samples were prepared by protein precipitation and subjected to quantitative analysis by LC-MS/MS using compound-specific mass transitions. Drug concentration-time profiles were generated and non-compartmental PK analysis used to generate estimates of half-life, clearance, volume of distribution and oral bioavailability.

### Results

In the above assays or similar assays, the compounds of formula (I) had an average pKi (pKb) at H3 of greater than about 8.5 and at H1 of less than about 5.7.
In addition, the compound of E1 showed an oral bioavailability of approximately 48%F in rat and of approximately 100%F in dog, with a CNS pentration of about 57.5 ng/g.

## Claims

1. A compound of formula (I) which is 4-[(2,4-difluorophenyl)carbonyl]-1-[4-({3-[2-methyl-1-pyrrolidinyl]propyl}oxy)phenyl]-2-piperazinone 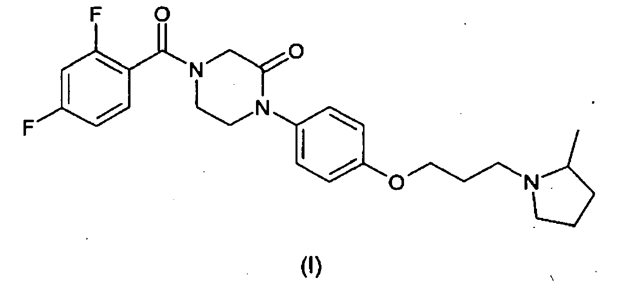 or a salt thereof.

2. Compound according to claim 1 wherein the salt is a pharmaceutically acceptable salt.

3. 4-[(2,4-Difluorophenyl)carbonyl]-1-[4-({3-[(2R)-2-methyl-1-pyrrolidinyl]propyl}oxy)phenyl]-2-piperazinone, or a salt thereof.

4. A process for the preparation of a compound of formula (I) or a salt thereof, the process comprising:
(a) reacting a compound of Formula (II) 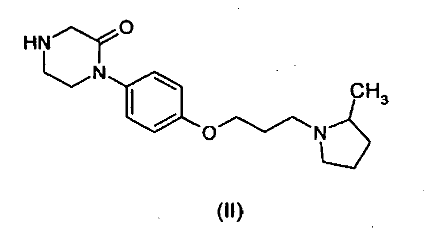 or a salt thereof, with 2,4-difluorobenzoic acid;
(b) isolating the individual R and S isomers from the racemate; or
(c) preparation of a salt of a compound of formula (I).

5. A compound or a pharmaceutically acceptable salt thereof according to any one of claims 1 to 3 for use in therapy.

6. A compound or a pharmaceutically acceptable salt thereof according to claim 5 for use in the treatment of inflammatory and/or allergic disorders.

7. A compound or a pharmaceutically acceptable salt thereof according to claim 5 for use in the treatment of rhinitis, particularly allergic rhinitis.

8. A composition which comprises a compound or a pharmaceutically acceptable salt thereof according to any one of claims 1 to 3 with one or more pharmaceutically acceptable carriers and/or excipients.

9. A composition according to claim 8 which further comprises an H1 receptor antagonist.

10. The use of a compound or a pharmaceutically acceptable salt thereof according to any one of claims 1 to 3 in the manufacture of a medicament for the treatment of inflammatory and/or allergic disorders.

11. The use according to claim 10 in which the disorder is allergic rhinitis.

## Patentansprüche

1. Verbindung der Formel (I), die 4-[(2,4-Difluorphenyl)carbonyl]-1-[4-({3-(2-methyl-1-pyrrolidinyl]propyl}oxy)phenyl]-2-piperazinon 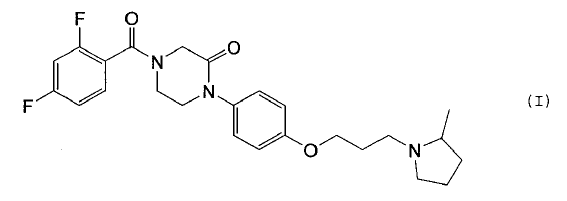 oder ein Salz davon ist.

2. Verbindung gemäß Anspruch 1, worin das Salz ein pharmazeutisch annehmbares Salz ist.

3. 4-[(2,4-Difluorphenyl)carbonyl]-1-[4-({3-[(2R)-2-methyl-1-pyrrolidinyl]propyl}oxy)phenyl]-2-piperazinon oder ein Salz davon.

4. Verfahren zum Herstellen einer Verbindung der Formel (I) oder eines Salzes davon, wobei das Verfahren umfaßt:
(a) Umsetzen einer Verbindung der Formel (II): 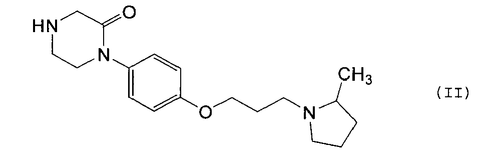 oder eines Salzes davon mit 2,4-Difluorbenzoesäure;
(b) Isolieren der einzelnen R- und S-Isomere aus dem Racemat; oder
(c) Herstellung eines Salzes einer Verbindung der Formel (I).

5. Verbindung oder pharmazeutisch annehmbares Salz davon gemäß einem der Ansprüche 1 bis 3 zur Verwendung in der Therapie.

6. Verbindung oder pharmazeutisch annehmbares Salz davon gemäß Anspruch 5 zur Verwendung in der Behandlung von entzündlichen und/oder allergischen Störungen.

7. Verbindung oder pharmazeutisch annehmbares Salz davon gemäß Anspruch 5 zur Verwendung in der Behandlung von Rhinitis, insbesondere allergischer Rhinitis.

8. Zusammensetzung, die eine Verbindung oder ein pharmazeutisch annehmbares Salz davon gemäß einem der Ansprüche 1 bis 3 mit einem oder mehreren pharmazeutisch annehmbaren Trägern und/oder Exzipienten umfaßt.

9. Zusammensetzung gemäß Anspruch 8, die ferner einen H1-Rezeptor-Antagonisten umfaßt.

10. Verwendung einer Verbindung oder eines pharmazeutisch annehmbaren Salzes davon gemäß einem der Ansprüche 1 bis 3 in der Herstellung eines Medikaments zur Behandlung von entzündlichen und/oder allergischen Störungen.

11. Verwendung gemäß Anspruch 10, worin die Störung allergische Rhinitis ist.

## Revendications

1. Composé de formule (I) qui est la 4-[(2,4-difluorophényl)carbonyl]-1-[4-({3-[2-méthyl-1-pyrrolidinyl]propyl}oxy)phényl]-2-pipérazinone 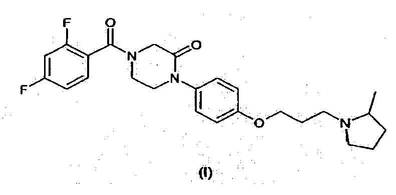 ou un sel de celui-ci.

2. Composé selon la revendication 1, où le sel est un sel pharmaceutiquement acceptable.

3. 4-[(2,4-Difluorophényl)carbonyl]-1-[4-({3-[(2R)-2-méthyl-1-pyrrolidinyl]propyl}oxy)phényl]-2-pipérazinone ou un sel de celle-ci.

4. Procédé de préparation d'un composé de formule (I) ou d'un sel de celui-ci, le procédé comprenant :
(a) la réaction d'un composé de formule (II) 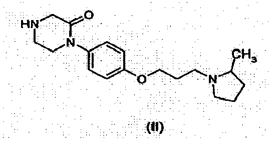 ou d'un sel de celui-ci, avec de l'acide 2,4-difluorobenzoïque ;
(b) l'isolement des isomères R et S individuels à partir du racémate ; ou
(c) la préparation d'un sel d'un composé de formule (I).

5. Composé ou sel pharmaceutiquement acceptable de celui-ci selon l'une quelconque des revendications 1 à 3 en vue d'une utilisation en thérapie.

6. Composé ou sel pharmaceutiquement acceptable de celui-ci selon la revendication 5 en vue d'une utilisation dans le traitement de troubles inflammatoires et/ou allergiques.

7. Composé ou sel pharmaceutiquement acceptable de celui-ci selon la revendication 5 en vue d'une utilisation dans le traitement de la rhinite, particulièrement la rhinite allergique.

8. Composition qui comprend un composé ou un sel pharmaceutiquement acceptable de celui-ci selon l'une quelconque des revendications 1 à 3 avec un ou plusieurs supports et/ou excipients pharmaceutiquement acceptables.

9. Composition selon la revendication 8 qui comprend en outre un antagoniste du récepteur H1.

10. Utilisation d'un composé ou d'un sel pharmaceutiquement acceptable de celui-ci selon l'une quelconque des revendications 1 à 3 dans la fabrication d'un médicament destiné au traitement de troubles inflammatoires et/ou allergiques.

11. Utilisation selon la revendication 10 où le trouble est la rhinite allergique.
